# EUROPEAN PATENT SPECIFICATION

(11) **EP 2 762 195 B1**
(45) Date of publication and mention of the grant of the patent: **09.09.2020**
(21) Application number: 14151887.8
(22) Date of filing: 21.01.2014
(51) Int. Cl.: A61B 18/00, A61N 7/00, A61B 17/22

(54) **Pressure-assisted irreversible electroporation**
Druckunterstützte irreversible Elektroporation
Électroporation irréversible assistée par pression

(30) Priority: 01.02.2013 US 201313756556
(43) Date of publication of application: 06.08.2014
(73) Proprietor: Moshe, Ein-Gal, 47203 Ramat Hasharon (IL)
(72) Inventor: Moshe, Ein-Gal, 47203 Ramat Hasharon (IL)
(74) Representative: Becker & Kurig Partnerschaft Patentanwälte PartmbB

(56) References cited:
- EP-A2- 2 425 871
- US-A- 6 041 253
- US-A1- 2007 095 648
- US-A1- 2010 191 235
- US-A1- 2010 233 021
- US-A1- 2012 046 658
- US-A1- 2012 157 891
- US-A1- 2012 220 998
- WHITNEY LONGSINE-PARKER ET AL: "Microfluidic electro-sonoporation: a multi-modal cell poration methodology through simultaneous application of electric field and ultrasonic wave", LAB ON A CHIP: MINIATURISATION FOR CHEMISTRY, PHYSICS, BIOLOGY, MATERIALS SCIENCE AND BIOENGINEERING, vol. 13, no. 11, 1 January 2013 (2013-01-01), page 2144, XP055354030, GB ISSN: 1473-0197, DOI: 10.1039/c3lc40877a

## Description

### FIELD OF THE INVENTION

The present invention generally relates to a system for treating tissue with combined electrical pulses and pressure waves.

### BACKGROUND OF THE INVENTION

US Patent 8282631 to Davalos et al. describes a minimally invasive surgical technique for tissue ablation, called irreversible electroporation.

As described in US Patent 8282631, electroporation is defined as the phenomenon that makes cell membranes permeable by exposing them to certain electric pulses. Electroporation pulses are defined as those electrical pulses that through a specific combination of amplitude, shape, time length and number of repeats produce no other substantial effect on biological cells than the permeabilization of the cell membrane.

The range of electrical parameters that produce electroporation is bounded by:
a) parameters that have no substantial effect on the cell and the cell membrane,
b) parameters that cause substantial thermal effects (Joule heating) and
c) parameters that affect the interior of the cell, e.g. the nucleus, without affecting the cell membrane.

Joule heating, the thermal effect that electrical currents produce when applied to biological materials, may be done by means of RF, ultrasound, laser light, electromagnetic induction, convection, mechanical stimulation and others. The pulse parameters that produce thermal effects are longer and/or have higher amplitudes than the electroporation pulses whose only substantial effect is to permeabilize the cell membrane.

Accordingly, electrical pulses that produce thermal effects are distinctly different from the pulses which produce electroporation. The effect of the thermal electrical pulses is primarily on the temperature of the biological material - the temperature is increased to induce tissue ablation through thermal effects. In contrast, the effect of the electroporation parameters is primarily on the cell membrane and their utility is in permeabilizing the cell membrane for various applications.

Electrical parameters that only affect the interior of the cell, without affecting the cell membrane were also identified recently. These pulses have high amplitude and short duration, which is substantially shorter than electroporation pulses, being in the range of nanoseconds. Such nanosecond pulses can affect the interior of the cell and in particular the nucleus without affecting the membrane.

Electrical pulses that produce intracellular effects are distinctly different from the pulses which produce electroporation. The effect of the intracellular electrical pulses is primarily on the intracellular contents of the cell and their utility is in manipulating the intracellular contents for various uses, e.g., ablation.

In electroporation, the permeabilization of the membrane can be reversible or irreversible as a function of the electrical parameters used. In reversible electroporation the cell membrane reseals a certain time after the pulses cease and the cell survives. In irreversible electroporation the cell membrane does not reseal and the cell lyses.

The electrical field for irreversible electroporation (IRE) of tissue is typically applied through electrodes, e.g., needle electrodes invasively placed around a cancer nodule, and skin electrodes non-invasively placed around a skin lesion.

Field concentration at the electrodes generally precludes treating internal tissue with non-invasive skin electrodes due to possible IRE and/or thermal damage to the skin.

It is important to note that US Patent 8282631 uses electrical pulses alone to induce irreversible electroporation.

Increase of membrane permeability is also possible by applying high pressure pulses to the cell membrane. Such pulses may increase permeability directly through cell deformation or indirectly through cavitation.

Tissue treatment by non-heating pressure waves is used for urinary stones disintegration (lithotripsy), pain alleviation in joints, skin treatment, revascularization, massage and others. Non-heating pressure waves include sub-ultrasonic waves and shockwaves. Sub-ultrasonic waves are pulses of sub-ultrasonic spectrum and sub-ultrasonic repetition rate. Typical shockwaves have a steep wave front followed by a shallower rarefaction tail. The tail may decay in oscillatory fashion. Tail rarefaction is associated with violent explosion of air bubbles in tissue or in liquid known as cavitation.

US2007/095648, US2010/191235, US2010/233021, US2012/046658, US 2012/220998 and US6041253 refer to additional documents relevant for the present invention. US2007/095648 is considered to be the closest prior art.

### SUMMARY OF THE INVENTION

The present invention is defined by the appended independent claim 1 and seeks to provide a novel system for pressure-assisted irreversible electroporation, by using a combination of electrical pulses and pressure waves, as is described more in detail hereinbelow. In contrast to US Patent 8282631, in the present invention, the electrical pulses without the pressure pulses are insufficient to induce irreversible electroporation of tissue cells; rather only the combination of electrical pulses and pressure waves is sufficient to induce irreversible electroporation.

There is thus provided in accordance with an example a method for pressure-assisted irreversible electroporation of tissue cells, including placing one or more electrodes near tissue cells to be treated, positioning a pressure source operable to apply pressure pulses to the tissue cells, and applying to the tissue cells a combination of electrical pulses through the one or more electrodes and pressure pulses from the pressure source such that the combination is sufficient to induce irreversible electroporation of the tissue cells, and wherein the electrical pulses without the pressure pulses are insufficient to induce irreversible electroporation of the tissue cells.

In accordance with a modification the method includes sensing characteristics of the tissue cells and using characteristics in a control loop to determine the combination.

In accordance with a modification the method includes adjusting an angle between an electrical field of the electrical pulses and a pressure propagation of the pressure pulses.

There is also provided in accordance with the present invention a system for pressure-assisted irreversible electroporation of tissue cells including an electric pulse generator operative to apply electrical pulses to one or more electrodes placed near tissue cells to be treated, a pressure source operative to apply pressure pulses to the tissue cells, and a controller in communication with the generator and the pressure source, the controller being operative to control delivery of electrical pulses through the one or more electrodes and pressure pulses from the pressure source such that the combination is sufficient to induce irreversible electroporation of the tissue cells, and wherein the electrical pulses without the pressure pulses are insufficient to induce irreversible electroporation of the tissue cells.

In accordance with an embodiment of the present invention a sensor is in communication with the controller, the sensor being operative to sense characteristics of the tissue cells, wherein the controller is operative to use the characteristics in determining the combination.

In accordance with an embodiment of the present invention an orientator is in communication with at least one of the generator and the pressure source, the orientator being operative to adjust an angle between an electrical field of the electrical pulses and a pressure propagation of the pressure pulses.

The pressure waves may include sub-ultrasonic pulses or shockwaves or a combination of both. The pressure waves may include extracorporeal or intracorporeal pressure waves or a combination of both. In accordance with the present invention the system focuses the pressure waves. Focusing is according to the shape of the treated tissue. The focal volume includes spherical, ellipsoidal-like or generally elongated shapes.

In accordance with an example the method includes localizing the target by producing images of the target and processing the images with respect to a reference to calculate a location of the target with respect to a coordinate system. Imaging may be done by ultrasound, x-ray, CT, MRI, optical or electrical imaging or any combination thereof.

### BRIEF DESCRIPTION OF THE DRAWINGS

The present invention will be understood and appreciated more fully from the following detailed description, taken in conjunction with the drawings in which:
Fig. 1 is a simplified illustration of a system for pressure-assisted irreversible electroporation of tissue, in accordance with an embodiment of the present invention; and
Fig. 2 is a simplified illustration of a method for pressure-assisted irreversible electroporation of tissue, in accordance with an example

### DETAILED DESCRIPTION OF EMBODIMENTS

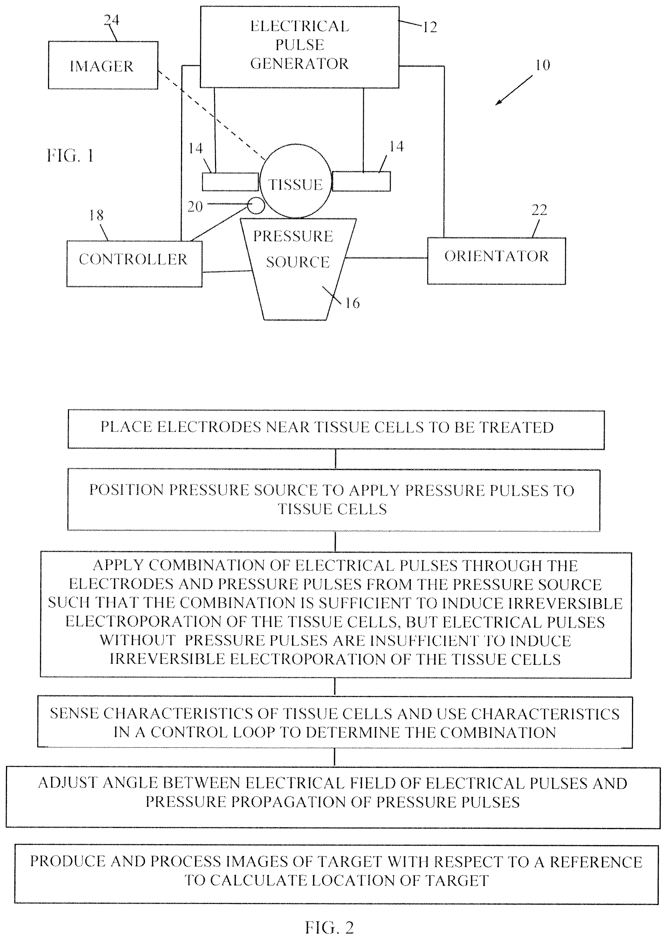

Reference is now made to Fig. 1, which illustrates a system 10 for irreversible electroporation of tissue, in accordance with a non-limiting embodiment of the present invention.

System 10 includes an electric pulse generator 12 operative to apply electrical pulses to one or more electrodes 14 placed near tissue cells to be treated.

Electrodes 14 may have different shapes and sizes and may be positioned at different distances from each other. The shape may be circular, oval, square, rectangular or irregular, and others. The distance of one electrode to another may be 0.5 to 10 cm, 1 to 5 cm, or 2-3 cm. The electrode may have a surface area of 0.1-5 cm² or 1-10 cm². The size, shape and distances of the electrodes can vary and the voltage and pulse duration used can also change accordingly.

A pressure source 16 is provided for applying pressure pulses or waves (the terms being used interchangeably) to the tissue cells. The non-heating pressure waves may include sub-ultrasonic waves and shockwaves or a combination of both. The pressure waves may include extracorporeal or intracorporeal pressure waves or a combination of both. The pressure waves are focused according to the shape of the treated tissue. The focal volume includes spherical, ellipsoidal-like or generally elongated shapes. Typical sub-ultrasonic waves are applied as pulses of sub-ultrasonic content and sub-ultrasonic repetition rate. Typical shockwaves have a steep wave front, followed by a shallower rarefaction tail that decays in oscillatory fashion. For example, extracorporeal shockwaves may be produced by electrohydraulic, electromagnetic or piezoelectric methods. Electrohydraulic shockwaves are formed with a high energy spark in water and an ellipsoidal reflector is used to focus the waves. Electromagnetic shockwaves are formed by producing a current pulse in a coil and inducing opposite current in an adjacent conducting membrane submerged in water. The repelling force of the opposing currents jerks the membrane and produces a wave. Focusing is by an acoustic lens, a reflector or by shaping a spherical membrane. Intracorporeal shockwaves may also be used, and may be produced by focusing laser light or creating a spark at the target.

A controller 18 is in communication with electric pulse generator 12 and pressure source 16. Controller 18 controls the operating parameters of electric pulse generator 12, such as pulse duration, voltage level, voltage gradient and others. Controller 18 controls the operating parameters of pressure source 16, such as pressure wave amplitude and frequency and others. Controller 18 uses its control of the operating parameters to create a combination of electrical pulses through electrode(s) 14 and pressure pulses from pressure source 16 such that the combination is sufficient to induce irreversible electroporation of the tissue cells; however, the electrical pulses without the pressure pulses are insufficient to induce irreversible electroporation of the tissue cells.

In the prior art US Patent 8282631, typical values for the electrical pulses for irreversible electroporation are as follows:
Pulse duration: in a range of from about 5 µsec to about 62000 msec, or about 75 µsec to about 20000 msec, or about 100 µsec ± 10 µsec.

Pulse voltage, that is, voltage gradient (voltage per centimeter): in a range of about 100 V/cm to 7000 V/cm, or 200 V/cm to 2000 V/cm, or 300 V/cm to 1000 V/cm, or about 600 V/cm ± 10%.

Irreversible electroporation is obtained by various combinations of electrical pulse duration, pulse voltage, pulse repletion rate and number of pulses. The collection of such combinations is referred to as the IRE electrical envelope.

The present invention introduces three additional variables, i.e., pressure pulse magnitude (in the range of about 20-500 Pa), pressure pulse duration (in the range of about 0.1 - 10 microseconds for supersonic waves) and angular deviation of the pressure propagation direction from the electrical field (in the range of 0-360 degrees).

IRE is then obtained by synchronizing pressure pulses and electrical pulses combination, wherein the electrical pulses combination is outside the IRE electrical envelope. Such electrical extra-envelope combinations may include pulse duration and pulse voltage inside the electrical IRE envelope (for example, pulses specified by US patent 8282631), provided that the associated pulse repetition rate and number of pulses will not render the combination as an adequate one for causing IRE (unlike US Patent 8282631). Another example for such an electrical extra-envelope combination is a combination similar to one in the IRE electrical envelope but with a lower pulse voltage.

In accordance with an embodiment of the present invention, one or more sensors 20 are in communication with controller 18. Sensors 20 may include, without limitation, a temperature sensor (e.g., thermistor or thermocouple), tissue capacitance sensor, tissue resistivity sensor and others. Sensor 20 senses characteristics of the tissue cells, such as but not limited to, temperature, heat dissipation capacity, thermal conductivity, specific heat, moisture and others. Controller 18 uses these characteristics in determining the combination of electrical pulses through electrode(s) 14 and pressure pulses from pressure source 16 such that the combination is sufficient to induce irreversible electroporation of the tissue cells.

In accordance with an embodiment of the present invention an orientator 22 is in communication with generator 12 and/or pressure source 16. Orientator 22 may be a turntable, linear actuator, and the like. Orientator 22 can adjust an angle between an electrical field of the electrical pulses and a pressure propagation of the pressure pulses. In this manner the treatment plan can be adjusted for any particular application.

The target tissue may be localized by producing images of the target with an imager 24 and processing the images with respect to a reference to calculate a location of the target with respect to a coordinate system. Imaging may be done by ultrasound, x-ray, CT, MRI, optical or electrical imaging or any combination thereof.

## Claims

1. A system (10) for pressure-assisted irreversible electroporation of tissue cells comprising:
an electric pulse generator (12) operative to apply electrical pulses to one or more electrodes (14) placed near tissue cells to be treated; and
a pressure source (16) operative to apply non-heating pressure pulses to said tissue cells; and a controller (18) in communication with said generator and said pressure source (16), which is operative to create a combination of electrical pulses through said one or more electrodes (14) and pressure pulses from said pressure source (16) such that the combination is sufficient to induce irreversible electroporation of said tissue cells, and **characterised**
**in that** said electrical pulses without said pressure pulses are insufficient to induce irreversible electroporation of said tissue cells, and
wherein the pressure waves are focused according to the shape of the treated tissue, the focal volume including spherical, ellipsoidal-like or generally elongated shapes.

2. The system (10) according to claim 1, wherein said pressure pulses comprise at least one of sub-ultrasonic pulses and shockwaves.

3. The system (10) according to claim 1, further comprising a sensor (20) in communication with said controller (18), the sensor (20) being operative to sense characteristics of said tissue cells, wherein said controller (18) is operative to use said characteristics in determining said combination.

4. The system (10) according to claim 1, further comprising an orientator (22) in communication with at least one of said generator (12) and said pressure source (16), said orientator (22) being operative to adjust an angle between an electrical field of said electrical pulses and a pressure propagation of said pressure pulses.

## Patentansprüche

1. System (10) für druckunterstützte irreversible Elektroporation von Gewebezellen, umfassend:
einen elektrischen Impulsgenerator (12), der betriebsfähig ist, elektrische Impulse an eine oder mehrere Elektroden (14) anzulegen, platziert in der Nähe von zu behandelnden Gewebezellen; und
eine Druckquelle (16), die betriebsfähig ist, um nicht erhitzende Druckimpulse an die Gewebezellen anzulegen; und
eine Steuerung (18) in Kommunikation mit dem Generator und der Druckquelle (16), die betriebsfähig ist, eine Kombination elektrischer Impulse durch die eine oder mehreren Elektroden (14) und Druckimpulse von der Druckquelle (16) zu erzeugen, so dass die Kombination ausreichend ist, um irreversible Elektroporation der Gewebezellen zu erzeugen, und
**gekennzeichnet dadurch, dass** die elektrischen Impulse ohne die Druckimpulse nicht ausreichend sind, um irreversible Elektroporation der Gewebezellen zu erzeugen, und
wobei die Druckwellen konzentriert sind gemäß der Form des behandelten Gewebes, wobei das betreffende Volumen sphärische, ellipsenförmig-artige oder generell längliche Formen umfasst.

2. System (10) nach Anspruch 1, wobei die Druckimpulse wenigstens eines von Sub-Ultraschallimpulsen und Stoßwellen umfassen.

3. System (10) nach Anspruch 1, ferner umfassend einen Sensor (20) in Kommunikation mit der Steuerung (18), wobei der Sensor (20) betriebsfähig ist, um Eigenschaften der Gewebezellen zu erfassen, wobei die Steuerung (18) betriebsfähig ist, die Eigenschaften zur Bestimmung der Kombination zu verwenden.

4. System (10) nach Anspruch 1, ferner umfassend einen Ausrichter (22) in Kommunikation mit wenigstens einem von dem Generator (12) und der Druckquelle (16), wobei der Ausrichter (22) betriebsfähig ist, um einen Winkel zwischen einem elektrischen Feld der elektrischen Impulse und einer Druckausbreitung der Druckimpulse einzustellen.

## Revendications

1. Un système (10) d'électroporation irréversible assistée par pression de cellules tissulaires, comprenant :
un générateur d'impulsions électriques (12) opérationnel pour appliquer des impulsions électriques à une ou plusieurs électrodes (14) placées près de cellules tissulaires à traiter ; et
une source de pression (16) opérationnelle pour appliquer des impulsions de pression non chauffantes auxdites cellules tissulaires ; et
un contrôleur (18) en communication avec ledit générateur et ladite source de pression (16), qui fonctionne pour créer une combinaison d'impulsions électriques à travers ladite une ou plusieurs électrodes (14) et des impulsions de pression provenant de ladite source de pression (16) de telle sorte que la combinaison soit suffisante pour induire une électroporation irréversible desdites cellules tissulaires, et **caractérisé en ce que** lesdites impulsions électriques sans lesdites impulsions de pression sont insuffisantes pour induire une électroporation irréversible desdites cellules tissulaires, et les ondes de pression sont focalisées en fonction de la forme du tissu traité, le volume focal comprenant des formes sphériques, de type ellipsoïdal ou généralement allongées.

2. Le système (10) selon la revendication 1, dans lequel lesdites impulsions de pression comprennent au moins des impulsions sous-ultrasoniques ou des ondes de choc, ou les deux.

3. Le système (10) selon la revendication 1, comprenant en outre un capteur (20) en communication avec ledit contrôleur (18), le capteur (20) étant opérationnel pour détecter les caractéristiques desdites cellules tissulaires, ledit contrôleur (18) étant opérationnel pour utiliser lesdites caractéristiques pour déterminer ladite combinaison.

4. Le système (10) selon la revendication 1, comprenant en outre un orienteur (22) en communication avec au moins un parmi ledit générateur (12) et ladite source de pression (16), ledit orienteur (22) étant opérationnel pour régler un angle entre un champ électrique desdites impulsions électriques et une propagation de pression desdites impulsions de pression.
